# EUROPEAN PATENT SPECIFICATION

(11) **EP 0 630 212 B1**
(45) Date of publication and mention of the grant of the patent: **08.07.1998**
(21) Application number: 93905832.7
(22) Date of filing: 08.02.1993
(51) Int. Cl.: A61B 17/15

(54) **SURGICAL CUTTING BLOCK**
CHIRURGISCHER SCHNEIDEBLOCK
BLOC COUPANT CHIRURGICAL

(30) Priority: 20.02.1992 US 838486
(43) Date of publication of application: 28.12.1994
(73) Proprietor: SYNVASIVE TECHNOLOGY, INC., El Dorado Hills, CA 95762 (US)
(72) Inventor: FISHER, Michael, G., Folsom, CA 95630 (US)
(74) Representative: Hall, Robert Leonard
(86) International application number: PCT/US93/01142
(87) International publication number: WO 93/16647

(56) References cited:
- US-A- 4 736 737
- US-A- 4 808 557
- US-A- 4 839 315
- US-A- 4 852 789
- US-A- 4 883 778
- US-A- 4 892 093
- US-A- 4 920 846
- US-A- 4 926 847
- US-A- 4 960 735
- US-A- 4 965 231
- US-A- 5 059 564
- US-A- 5 085 671
- US-A- 5 092 869

## Description

### Field of the Invention

The field of the invention relates to surgical devices, and more particularly to surgical cutting blocks used for guiding saws and similar cutting devices in the shaping of bone, cartilage and other medium to hard tissue.

### Reference

Sunderman et al. 1989. Journal of Orthopaedic Research 7:307-315.

### Background of the Invention

Artificial joints, such as knee and hip socket replacements, are frequently implanted in the body to repair or replace damaged or diseased joints. In order to achieve a successful implant, the bone adjacent to the joint must first be cut and shaped in an appropriate shape that is geometrically reciprocal to the shape of the implant. Typically, the majority of cutting is done with a saw blade attached to a motorized surgical handpiece, which propels the blade in a variety of directional or hi-directional motions.

In most joint replacement surgery, the fit between the bone surface and the replacement is very precise, often with tolerances of a few thousandths of 25.4 millimetres (an inch). Virtually all surgeons use a cutting block to force, hold captive or guide a blade along a reference surface along one or more sides of the cutting block. The reference surface assures that the cutting plane will be extended to and through the bone by helping to guide the blade on its path through the bone. A typical cutting block is described in US-A-4926847.

Although widely used, known cutting blocks and surgical methods based on the use of known cutting blocks suffer from several serious problems. A commonly encountered problem is systemic toxicity following treatment. Standard surgical cutting blocks are made from various grades of stainless steel that are quickly eroded by the high speeds at which most surgical blades operate. The result of such erosion is the production of a slurry, commonly referred to in the industry as "sludge," in and near the operation site. The sludge contains the various elements present within the alloy making up both the cutting block and the surgical blades. A number of metals often found in stainless steel alloys, including both nickel and chrome, are left behind in the joint and eventually make their way throughout the patient's body. Nickel in particular is a known carcinogen. In a recent study, Sunderman et al. (1989) report that nickel concentrations in patients having joint replacement surgery rose 11 fold in the 1-2 days following surgery as compared to preoperative levels.

Aside from the problem of toxic sludge, erosion of stainless steel cutting blocks quickly cause the fretting of the reference plane surface, thereby destroying the ability of the cutting block to provide a precise reference edge during surgery. In most applications, tolerances of a few thousandths of 25.4 millimetres (an inch) are lost after 5-10 minutes of cutting, thereby forcing the surgeon to replace the cutting block (often impractical during surgery) or accept a less precise cut. Unfortunately, the failure to provide a precise alignment along the surface of contact between the prosthesis and the remaining bone can result in post-operative bone degradation, infection and joint failure.

Another serious problem encountered in the use of known cutting blocks is the heat of friction created during surgery. Although much of the heat comes from the frictional interaction of the saw teeth and the bone, a substantial amount of heat is generated and thus added to the blade by friction between the blade and the cutting block. It is well known that damage to bone tissue begins after bone temperature exceeds 50C and that irreparable damage takes place after temperatures exceed 70C for three or more minutes. Existing cutting devices and methods of use can generate heat in excess of 50C and even 70C.

In view of the foregoing, there is a clear need for a cutting block that does not deliver toxic elements to the patient as a by-product of erosion of the cutting block and/or blade. There is also a need for a cutting block with superior hardness that is capable of retaining its original configuration without unacceptable fretting during surgery. A further need is for a cutting block and blade combination that has a relatively low coefficient of friction during operation, thereby reducing blade heating and bone tissue degradation.

US-A-4960735 discloses an alumina based ceramic composition comprising carbide whiskers, zirconia, a residue of magnesia and alumina, such composition being for use in cutting tools employed at temperatures between 600C and 1200C.

### Summary of the Invention

It is a general object of the invention to provide an improved cutting block having a reference surface composed of a composition that has minimal amounts of toxic and carcinogenic elements such as nickel and chrome.

It is another object of the present invention to provide a cutting block having a cutting guide surface with a superior hardness such that the configuration and tolerances of the cutting surface is retained substantially throughout use during an operation.

It is yet another object of the present invention to provide a cutting block having a cutting guide surface that has a relatively low coefficient of friction when used in surgery with a given blade whereby heat build up in the blade from frictional contact with the cutting guide surface is minimised.

The invention meets these objects by providing an improved cutting block having one or more cutting guide surfaces characterised in that it or they comprise a composition having a Knoop hardness of 466 or greater (under 500gm load or greater), a chrome content of less that 10% by weight and a nickel content that is substantially less than 4% by weight.

The cutting block of the invention may be entirely composed of the desired composition or may be a composite construction having a core unit composed of stainless steel or other material suitable for surgical applications that is fitted with and coupled to one or more units composed of the composition described above and configured with a desired cutting guide surface. Such units may be discrete blocks or laminas that are physically affixed to or inlaid into a desired surface of the core unit or alternatively may constitute coatings or deposits that are bonded to a desired surface of the core unit using known techniques.

The surgical device of the invention are advantageous over prior art in that toxic deposits within the joint as a result of surgery are minimized, in that the tolerances of the reference cutting surface are maintained throughout surgery, and in that blade heating due to friction between the blade and cutting guide surface is reduced.

These and other objects and advantages of the invention will become more fully apparent when the following detailed description of the invention is read in conjunction with the accompanying drawings.

### Brief Description of the Drawings

FIGS. 1A & 1B are views in perspective of two embodiments of the cutting block of the invention.

FIG. 2 is a view in perspective of a first saw-captive embodiment of the cutter of the invention.

FIG. 3 is a view in perspective of a second saw-captive embodiment of the cutter of the invention.

### Detailed Description of the Invention

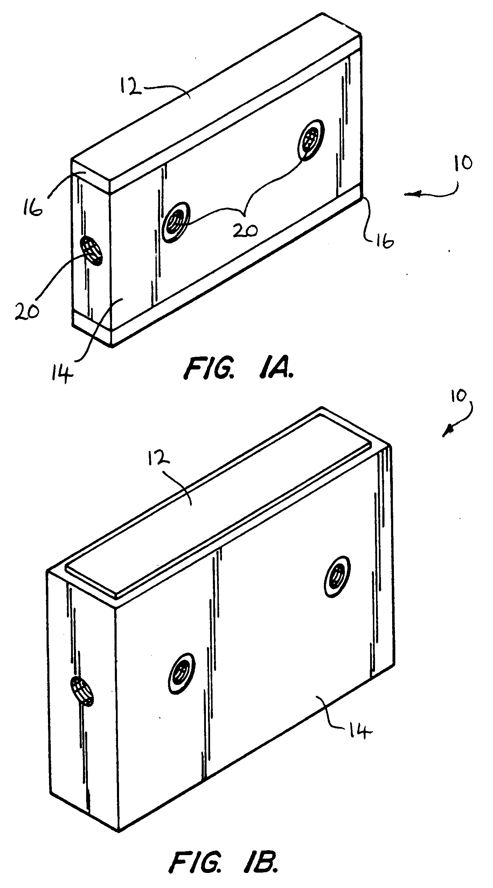

Turning now to the figures, the cutting block of the invention will now be described. According to one aspect of the invention, cutting block **10** can be comprised of a single material or composition that has been shaped to provide one or more cutting guide surfaces **12**. Alternatively, as shown in FIG. 1A and 1B, cutting block **10** may be comprised of a core unit **14** coupled to one or more cutting guide units **16**. Additionally, cutting block **10** can be provided with positioning pins (not shown) that are either integral with the cutting block or which may be attached to the cutting block via screw holes **20**.

Although the composition of the interior portion of the block and positioning pins can be of almost any durable, hard material that is not easily fractured, such as stainless steel, the cutting guide surface **12** of the cutting block should be of a material having a high degree of hardness coupled with low chrome and nickel content. Satisfactory parameters for such a material include a Knoop hardness of 466 or greater (under a 500 gm load or greater), a chrome content of less than 10% and a nickel content of less than 4%. A preferred material would have a Knoop hardness of 800 or greater (under a 500 gm load or greater), a chrome content of less than 8% and a nickel content of less than 3%. A most preferred material would have a Knoop hardness of 1000 or greater (under a 500 gm load or greater), a chrome content of less than 6% and a nickel content of less than 2%.

It will be appreciated that the cutting guide units **16** can be laminated or otherwise affixed at desired positions and angles on a core unit that may or may not have these characteristics. Alternatively, such cutting surface may be achieved by directly depositing a material having these characteristics on a preformed cutting guide surface, so long as the thickness of the material deposited is sufficiently thick to perform under normal surgical conditions. Of course, it will also be apparent to one skilled in the art that the entire block may be fabricated out of a composition having the desired hardness and chemical composition outlined above, provided that doing so is time and cost effective.

Materials having the desired characteristics that would be suitable for this work include ceramics such as zirconia, aluminas, certain borides such as titanium diboride and boron carbide, nitrogen-hardened titanium and similar materials. Such materials are known in the art and can be obtained from Coors Ceramic Company (Golden, Colorado). Although any of the materials just listed have the desired characteristics for cutting surface material as described above, ceramics like zirconia with very high Knoop hardness, high fracture toughness and low thermal conductivity are preferred. Such compounds are additionally advantageous because they have low coefficients of friction when used with standard metallic cutting blades, thus reducing heat generation, and are essentially nickel and chrome-free.

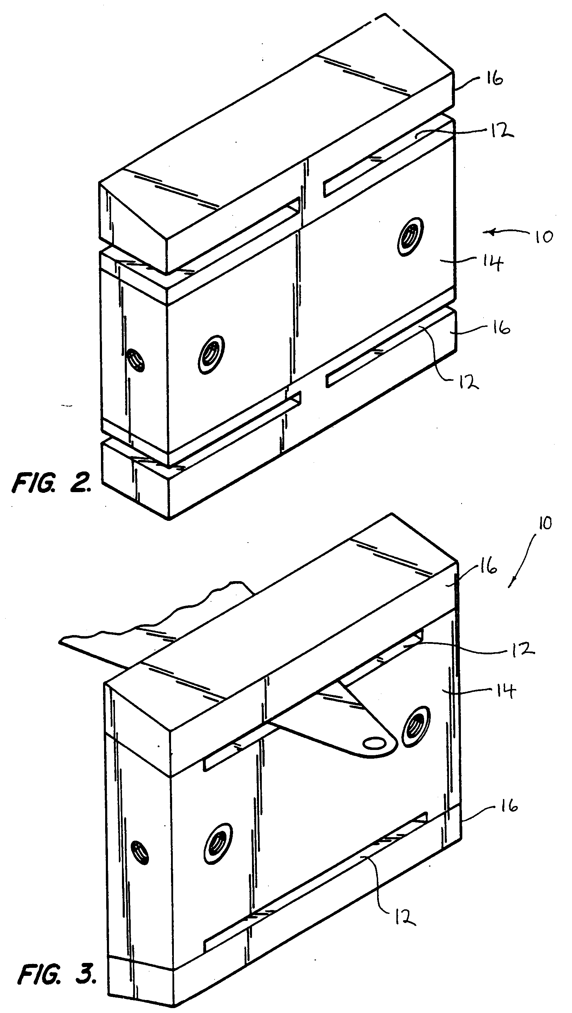

Although cutting blocks with cutting guide surfaces positioned on one or more outside faces are suitable for all types of orthopaedic surgery, saw-captive blocks are preferred by some surgeons. Saw-captive embodiments of the cutting block of the invention are depicted in FIGS. 2 and 3. Cutting block **10** may be configured to provide open channels extending laterally from each end, as shown in FIG. 2 or may be provided with internal channels as shown in FIG. 3. In each case, the block may be comprised of a solid piece of material having the desired hardness and metal content characteristics, or may be a composite comprised of a core unit **14** coupled with cutting units **16** so that the cutting guide surfaces **12** are comprised of the desired material.

In typical use, a surgeon will first expose the joint or joint region to be replaced. Holes will be drilled into the bone having a depth and position reciprocal to the configuration of the alignment pins of the cutting block so that the cutting guide surface of the cutting block is properly aligned to cut the bone at a desired angle and to a desired depth using a standard saw. A cutting block having a cutting guide surface composed of a material having the hardness and chemical characteristics set forth above is then implaced into the joint by inserting the alignment pins into the bone drill holes. The surgeon then uses the cutting guide surface to align and guide the blade as a cut is made into the cartilage and bone tissue. With cutting blocks having the cutting guide surface on an external face of the block, the surgeon may place pressure on the blade during surgery to cause the blade to bow, thereby adjusting the angle of the cut as surgery proceeds. After the bone has been cut and removed, the joint prothesis is implaced and the joint is closed. The cutting block of the invention may also be used to cut soft tissues, either using adjacent bone as a reference anchor or by using a adjustable mount separate from the patient.

From the foregoing, it will be appreciated how the objects and features of the invention are met. The hardness of the cutting guide surface helps to maintain an even reference cutting surface that is not liable to fret. The preferred materials are also low in toxic metal ions which may be shed in residue during surgery. Further, the hardness of the material, particularly ceramics such as zirconia, ensure that fretting is minimized which reduces friction and thus heat generation during operation and reduces sludge production that can be shed during the operation in the treatment area.

Although the invention has been described with respect to a particular surgical cutting block, it will be appreciated that various modifications of the apparatus and method are possible without departing from the invention, which is defined by the claims set forth below.

## Claims

1. A surgical cutting block (10) having at least one cutting guide surface (12) for guiding bone saws and similar blades characterised in that the cutting guide surface is comprised of a material having a Knoop hardness of 466 or greater, under a 500gm load or greater, a chrome content of 10% or less, and a nickel content of less than 4%.

2. The surgical cutting block of claim 1 characterised in that the material is selected from the group consisting of zirconia, alumina, nitrogen-hardened titanium, and borides.

3. The surgical cutting block of claim 2 characterised in that the material is zirconia.

4. The surgical cutting block of any of claims 1 to 3, characterised in that, it further comprises:
a) a core unit (14); and
b) at least one cutting guide unit (16) coupled to said core unit and comprised of said material.

5. The surgical cutting block of any of claims 1 to 3, characterised in that, it further comprises:
a) a core unit having a first end and a second end; and
b) a first cutting guide unit coupled to the first end of said core unit and provided with a through channel having at least one of said cutting guide surfaces; and
c) a second cutting guide unit coupled to the second end of said core unit and provided with a through channel having at least another of said cutting guide surfaces,
wherein said first and second cutting guide units are comprised of said material.

## Patentansprüche

1. Chirurgischer Schneidblock (10) mit wenigstens einer Schneidführungsfläche (12) zum Führen von Knochensägen und ähnlichen Blättern, dadurch gekennzeichnet, daß die Schneidführungsfläche ein Material mit einer Knoop-Härte von 466 oder mehr unter einer Last von 500 gm oder mehr, einem Chromgehalt von 10% oder weniger und einem Nickelgehalt von weniger als 4% aufweist.

2. Chirurgischer Schneidblock nach Anspruch 1, dadurch gekennzeichnet, daß das Material ausgewählt ist aus der Gruppe, die aus Zirkonoxid, Aluminiumoxid, stickstoffgehärtetem Titan und Boriden besteht.

3. Chirurgischer Schneidblock nach Anspruch 2, dadurch gekennzeichnet, daß das Material Zirkonoxid ist.

4. Chirurgischer Schneidblock nach einem der Ansprüche 1 bis 3, dadurch gekennzeichnet, daß er ferner aufweist:
a) eine Kerneinheit (14) und
b) wenigstens eine Schneidführungseinheit (16), die mit der Kerneinheit gekoppelt ist und das genannte Material aufweist.

5. Chirurgischer Schneidblock nach einem der Ansprüche 1 bis 3, dadurch gekennzeichnet, daß er ferner aufweist:
a) eine Kerneinheit mit einem ersten Ende und einem zweiten Ende; und
b) eine erste Schneidführungseinheit, die mit dem ersten Ende der Kerneinheit gekoppelt ist und mit einem Durchgangskanal versehen ist, der wenigstens eine der Schneidführungsflächen hat; und
c) eine zweite Schneidführungseinheit, die mit dem zweiten Ende der Kerneinheit gekoppelt ist und mit einem Durchgangskanal versehen ist, der wenigstens eine andere der Schneidführungsflächen hat,
wobei die erste Schneidführungseinheit und die zweite Schneidführungseinheit das genannte Material aufweisen.

## Revendications

1. Bloc de coupe chirurgicale (10) comportant au moins une surface de guide de coupe (12) pour guider les scies à os et des lames similaires, caractérisé en ce que la surface du guide de coupe est constituée d'un matériau ayant une dureté Knoop de 466 ou davantage, sous une charge de 500mg ou davantage, et une teneur en chrome de 10% ou moins, et une teneur en nickel de moins de 4%.

2. Bloc de coupe chirurgicale selon la revendication 1, caractérisé en ce que le matériau est choisi dans le groupe constitué de zircone, d'alumine, de titane trempé à l'azote, et de borures.

3. Bloc de coupe chirurgicale selon la revendication 2, caractérisé en ce que le matériau est de la zircone.

4. Bloc de coupe chirurgicale selon l'une des revendications 1 à 3, caractérisé en ce qu'il comprend en outre :
a) une unité de base (14) et
b) au moins une unité de guidage de coupe (16) accouplée à l'unité de base et constituée dudit matériau.

5. Bloc de coupe chirurgicale selon l'une des revendications 1 à 3, caractérisé en ce qu'il comprend en outre :
a) une unité de base ayant une première extrémité et une seconde extrémité, et
b) une première unité de guide de coupe accouplée à ladite première extrémité de l'unité de base et pourvue d'un canal traversant ayant au moins l'une des dites surfaces de guide de coupe, et
c) une seconde unité de guide de coupe couplée à la seconde extrémité de l'unité de base et munie d'un canal traversant ayant au moins une autre des dites surfaces de guide de coupe, dans lequel les dites premières et secondes unités de guide de coupe sont constituées dudit matériau.
